# EUROPÄISCHE PATENTSCHRIFT

(11) **EP 0 451 236 B1**
(45) Veröffentlichungstag und Bekanntmachung des Hinweises auf die Patenterteilung: **13.09.1995**
(21) Anmeldenummer: 90915081.5
(22) Anmeldetag: 05.10.1990
(51) Int. Cl.: B01D 61/36, A61K 9/127

(54) **VERFAHREN ZUR HERSTELLUNG VON WÄSSRIGEN DISPERSIONEN**
PROCESS FOR MAKING AQUEOUS DISPERSIONS
PROCEDE DE PREPARATION DE DISPERSIONS AQUEUSES

(30) Priorität: 13.10.1989 DE 3934656
(43) Veröffentlichungstag der Anmeldung: 16.10.1991
(73) Patentinhaber: SCHERING AKTIENGESELLSCHAFT, 13342 Berlin (DE)
(72) Erfinder: RÖSSLING, Georg, D-1000 Berlin 28 (DE); SACHSE, Andreas, D-1000 Berlin 10 (DE)
(86) Internationale Anmeldenummer: DE9000767
(87) Internationale Veröffentlichungsnummer: WO9105599

(56) Entgegenhaltungen:
- EP-A- 0 240 803
- Proceedings of the Annual International Conference of the IEEE Engineering in Medicine and Biology Society, Part 1/4: Cardiology and Imaging, 4-7 November 1988, New Orleans, Louisiana, US, Band 10, IEEE, M.Capellier et al.: "Radiopaque liposomes for computed tomography opacification", Seiten 491-492
- Derwent File Supplier WPIL, 1983, AN 83-707627 (28), Derwent Publications Ltd, (London, GB), & JP-A-58092408 (ASAHI GLASS K.K.) 1. Juni 1983
- Derwent File Supplier WPIL, 1983, AN 83-707626 (28), Derwent Publications Ltd, (London, GB), & JP-A-58092407 (ASAHI GLASS K.K.) 1. Juni 1983
- Derwent File Supplier WPIL, 1983, AN 83-707623 (28), Derwent Publications Ltd, (London, GB), & JP-A-58092404 (ASAHI GLASS K.K.) 1. Juni 1983
- Derwent File Supplier WPIL, 1989, AN 89-304166 (42), Derwent Publications Ltd, (London, GB), & JP-A-1224042 (NIPPON LEDERLE K.K.) 7. September 1989

## Beschreibung

Die Erfindung betrifft ein Verfahren zur Herstellung einer Liposomen oder Micellen enthaltenden wässrigen Dispersion, indem man die Liposomen und/oder Micellen bildenden Substanzen und gegebenenfalls in Wasser schwer lösliche oder unlösliche Wirkstoffe in einem organischen Lösungsmittel löst, die Lösung in eine wässrige Phase einträgt und anschließend das organische Lösungsmittel destillativ entfernt, welches dadurch gekennzeichnet ist, daß die destillative Entfernung des organischen Lösungsmittels mittels Membrandestillation erfolgt.

Organische Lösungsmittel, die mittels des erfindungsgemäßen Verfahrens abgetrennt werden können sind vorzugsweise solche, deren Siedepunkt bei maximal 300°C liegt. Derartige Lösungsmittel sind beispielsweise Alkohole, wie Methanol, Ethanol, Propanol oder Isopropanol, Ketone wie Aceton, Ester wie Ethylacetat, Ether wie Diethylether, Diisopropylether, Dioxan oder Tetrahydroduran, halogenierte Kohlenwasserstoffe wie Dichlormethan, Trichlorfluormethan, Kohlenwasserstoffe wie Pentan, Hexan, Heptan, Oktan, Neopentan, Isopentan, Cyclopentan oder polare aproptische Lösungsmittel wie Acetonitril, Dimethylsulfoxid oder Dimethylformamid.

Das erfindungsgemäße Verfahren ist selbstverständlich auch dazu geeignet, Gemische der obengenannten Flüssigkeiten abzutrennen. Das erfindungsgemäße Verfahren wird vorzugsweise in der Weise durchgeführt, daß man die Flüssigkeiten mittels transmembraner Destillation oder Pervaporation entfernt.

Liposomen oder Micellen enthaltende, wässrige Phasen werden häufig in der Weise so hergestellt, daß man die Liposomen- oder Micellen bildenden Substanzen und gegebenenfalls auch die Wirkstoffe in einem Lösungsmittel löst, die Lösung in die gegebenenfalls arzneimittelhaltige wässrige Phase einträgt, und gegebenenfalls nach Homogenisierung das Lösungsmittel destillativ entfernt (Pharmazie in unserer Zeit, 11, 1982, 97-108; Pure and Appl. Chem., 53, 1981, 2241-2254; DE-A 27 30 570).

Bei der Herstellung derartiger wässriger Dispersionen erzielt man überraschenderweise in der Regel Dispersionen mit in weiten Grenzen frei einstellbaren Teilchengrößen und signifikant gleichmäßigere Partikelgrößen-Verteilung wenn man die zu entfernende Flüssigkeit nicht einfach destillativ, sondern mittels Membrandestillation entfernt. Darüberhinaus hat das erfindungsgemäße Verfahren insbesondere bei der Herstellung von Liposomen oder Micellen enthaltenden Phasengemischen den Vorzug, daß es im technischen Maßstabe wesentlich einfacher durchführbar ist als beispielsweise das REV-Verfahren, welches bekanntlich zur technischen Herstellung derartiger Dispersionen wenig geeignet ist.

Unter dem Begriff Membrandestillation sollen erfindungsgemäß insbesondere die bekannten Verfahren der transmembranen Destillation (Chem. Ing. Techn. 56, 1984, 514-521; J. of Membrane Sci., 39, 1988, 25-42; DE-A 33 12 359) und Pervaporation (Swiss Chem. 10, 1988, 45-51; ACS Symposium 281, 1985, 467-478; Chem. Ing. Tech. 60, 1988, 590-603) und der Pervaporation verstanden werden.

Bei der transmembranen Destillation (TMD), welche technisch bereits bei der Aufreinigung von Wasser und in der Lebensmittelindustrie Anwendung findet wird die zu entfernende Flüssigkeit bekanntlich über eine hydrophobe, symmetrische mikroporöse Membran entfernt. Geeignete Membrane sind beispielsweise solche aus Polyolefinen wie Polypropylen und Polyfluorkohlenwasserstoffen wie Polytetrafluorethylen und Polyvinylidenfluorid mit einer Porengröße von 0,1-0,5µm, die zu Kapillaren von ca. 1-2 mm Durchmesser und etwa 0,5 bis 1 mm Wandstärke geformt sind. Die transmembrane Destillation wird in der Regel mit Filtrationsmodulen durchgeführt, welche die entsprechenden Membrane in Rohr- oder Kapillarform enthalten. Zur Erzielung eines ausreichenden Permeatflusses sollte die Temperaturdifferenz zwischen der im Innenraum der Kapillaren befindlichen wässrigen Dispersion und dem außerhalb der Kapillaren befindlichen Permeat auf einem Bereich von ca. 1° C bis 100° C und vorzugsweise 10° bis 60° C eingestellt werden. Des weiteren ist zur Erzielung eines ausreichenden Membranflusses das Anlegen eines Druckgradienten geeignet. Die Größe des Druckes richtet sich nach der Druckfestigkeit der verwendeten Membrane und beträgt in der Regel maximal 10⁶PA. Die transmembrane Destillation eignet sich sowohl zum Entfernen flüchtiger organischer Lösungsmittel mit einem höheren Dampfdruck als Wasser aus den Flüssigkeitsgemischen, als auch zur Konzentrierung der erhaltenen Dispersionen.

Bei der Pervaporation, welche technisch bereits zur Entfernung von Ethanol aus Fermentationsbrühen Anwendung findet, wird die zu entfernende Flüssigkeit bekanntlich über eine asymmetrische Membran entfernt, die keine Poren hat. Geeignete Membranen sind beispielsweise solche aus Polydimethylsiloxan oder Polyvinylalkohol von etwa 0,1 bis 2 µm Stärke die auf einer schwammartigen oder gewebsartigen Stützschicht aufgebracht sind. Geeignete Membranmodule sind ebenfalls Kapillar- und Rohrmodule oder auch Plattenmodule oder Spiralwickelmodule. Bezüglich der Entwicklung lösungsmittelselektiver Membranen und ihrer Wirkungsweise sei auf die bereits erwähnte Publikation in der Zeitschrift Chem. Ing. Techn. 60, 1988, 590 ff verwiesen.

Das Pervaporationsverfahren kann nicht nur dazu verwendet werden, solche Lösungsmittel aus wässrigen Dispersionen zu entfernen, die einen höheren Dampfdruck als Wasser haben. sondern eignet sich auch zur Entfernung von Lösungsmitteln mit einem niedrigeren Dampfdruck als Wasser, wie beispielsweise Dimethylformamid. Dimethylsulfoxid oder Acetonitril.

In Wasser schwer lösliche oder unlösliche pharmazeutische Wirkstoffe folgender Wirkstoffgruppen sind gegebenenfalls bei der Herstellung der wässrigen Dispersionen nach dem erfindungsgemäßen Verfahren in der Lösung enthalten.

Gestagen wirksame Steroidhormone wie beispielsweise das 13-Ethyl-17β-hydroxy 18, 19-dinor-17α-pregn-4-en-20yl-3-on (=Levonorgestrel), das 13-Ethyl-17β-hydroxy-18, 19-dinor-17α-pregna-4,15-dien-20yn-3-on (=Gestoden) oder das 13-Ethyl17β-hydroxy-11-methylen-18,19-dinor-17α-pregn-4-en-20yn (Desorgestrel), Estrogen wirksame Steroidhormone wie 3-Hydroxy-1,3,5(10)-estratrien-17-on (=Ostron) oder 1,9-Nor-17α-pregna-1,3,5(10)-trien-20-yn-3,17β-diol (Ethinylöstradiol).

Androgen wirksame Steroidhormone wie 17β-Hydroxy-4-androsten-3-on (=Testosteron) und dessen Ester oder 17β-Hydroxy-1α-methyl-5α-androsten-3-on (=Mesterolon).

Antiandrogen wirksame Steroidhormone wie das 17α-Acetoxy-6-chlor-1β,2β-dihydro-3 H-cyclopropa[1,2]-pregna-1,4,6-trien-3,20-dion (Cypoteronacetat).

Kortikoide wie das 11β, 17α, 21-Trihydroxy-4-pregnen-3,20-dion (=Hydrocortison), das 11β, 17α,21-Trihydroxy-1,4-pregnadien-3,20-dion (=Prednisolon), das 11β, 17α-21-Trihydroxy-6α-methyl-1,4-pregnatrien-3,20-dion - (=Methylprednisolon) und das 6α-Fluor-11β,21-dihydroxy-16α-methyl-1,4-pregnadien-3,20-dion (=Difluocortolon) und deren Ester.

Ergoline wie der 3-(9,10-Dihydro-6-methyl-8α-ergolinyl)-1,1-diethylharnstoff (=Ergolin), der 3-(2-Brom-9,10-dihydro-6-methyl-8α-ergolinyl)-1,1-diethylharnstoff (=Bromergolin) oder der 3-(6-Methyl-8α-ergolinyl)-1,1-diethylharnstoff (=Tergurid).

Antihypertonika wie das 7α-Acetylthio-17α-hydroxy-3-oxo-4-pregnen-21-carbonsäure-γ-lacton (=Spironolacton) oder das 7α-Acetylthio-15β, 16β-methylen-3-oxo-17α-pregna-1,4-dien-21,17-carbolacton (=Mespirenon).

Antikoagulantia wie die 5-[Hexahydro-5-hydroxy-4-(3-hydroxy-4-methyl-1-octen-6-ynyl)-2(1H)-pentalenyliden)]-pentansäure (=Iloprost).

Psychopharmaka wie das 4-(3-Cyclopentyloxy-4-methoxy-phenyl-2-pyrrolidon (=Rolipram) und das 7-Chlor-1,3-dihydro-1-methyl-5-phenyl-2H-1,4-benzodiazepin-2-on (=Diazepam).

Carotinoide wie das α-Carotin und das β-Carotin.

Fettlösliche Vitamine, wie Vitamine der Vitamin A-, Vitamin D-, Vitamin E- und Vitamin K-Gruppe.

Eine weitere Gruppe sind β-Carboline, wie sie beispielsweise in den Europäischen Patentanmeldungen 234,173 und 239,667 beschrieben sind. Als β-Carboline seien beispielsweise genannte der 6-Benzoyloxy-4-methoxymethyl-βcarbolin-3-carbonsäure-isopropylester (=Becarnil) und der 5-(4-Chlorphenoxy)-4-methoxymethyl-β-carbolin-3-carbonsäure-isopropylester (=Cl-PHOCIP).

Erwähnenswert sind auch schwerlösliche Kontrastmittel, wie das Röntgenkontrastmittel Iodipamidethylester oder NMR-Kontrastmittel wie die Eisen- oder Manganporphyrinchelate.

Besondere Vorteile bietet das erfindungsgemäße Verfahren bei der Herstellung von Liposomen oder Micellen enthaltenden Phasengemischen im technischen Maßstab, da sich diese mittels der vorbekannten Methoden nur schwierig in größeren Mengen herstellen lassen, wie bereits erwähnt wurde.

Liposomen oder Micellen enthaltende Phasengemische sind bekanntlich unter anderem zur Verkapselung oder Solubilisierung von Wirkstoffen von Bedeutung. Erfindungsgemäß werden sie in der Weise hergestellt, daß man die Liposomen und/oder Micellen bildenden Substanzen und gegebenenfalls auch die Wirkstoffe in einem flüchtigen organischen Lösungsmittel (wie zum Beispiel Ethanol, Ethylacetat, Diethylether) löst, die Lösung in die gegebenenfalls wirkstoffhaltige wässrige Phase einträgt und das Lösungsmittel durch transmembrane Destillation oder Pervaporation entfernt.

Geeignete Micellen bildende Substanzen sind insbesondere Salze von Gallensäuren, welche in Kombination mit Lipiden zur Herstellung wässriger Mischmicellenlösungen verwendet werden. (DE-A 27 30 570).

Als geeignete Gallensäuren seien beispielsweise genannt: Die Cholsäure, die Glycocholsäure, die Taurocholsäure, die Deoxycholsäure, die Glycodeoxycholsäure, die Taurodeoxycholsäure, die Chenodeoxycholsäure, die Glycochenodeoxycholsäure und die Taurochenodeoxycholsäure.

Zur Herstellung der wässrigen Mischmicellösungen können bei dem erfindungsgemäßen Verfahren die gleichen Lipide verwendet werden, wie bei den vorbekannten Verfahren.

Geeignete Lipide sind beispielsweise, Monoglyceride, Sulfatide, und insbesondere Phospholipide, wie die Sphingomyeline, die Plasmalogene, die Phosphatidylcholine, die Phosphatidylethanolamine, die Phosphatidylserine, die Phosphatidylinosite und die Cardiolipine und auch Gemische dieser Lipide (Dr. Otto-Albert Neumüller: Römpps Chemie-Lexikon; Franck'sche Verlagshandlung, Stuttgart(DE) 2665, 3159, 3920 und 4045).

Zur Herstellung der wässrigen Mischmicellösungen werden vorzugsweise 3 bis 40 % und insbesondere 5 bis 20 % Lipid pro 100 g der gegebenenfalls isotonisierende Zusätze und/oder wasserlösliche Wirkstoffe enthaltenden wässrigen Lösung verwendet. Das Gewichtsverhältnis zwischen Lipid und Gallensäure beträgt vorzugsweise 0,1:1 bis 2:1 und insbesondere 0,8:1 bis 2:1.

Als Basen zur Herstellung der Salze der Gallensäuren eignen sich beispielsweise Alkalihydroxide, wie Lithiumhydroxid, Kaliumhydroxid und insbesondere auch Natriumhydroxid.

Die nach dem erfindungsgemäßen Verfahren hergestellten wässrigen Mischmicellösungen können gewünschtenfalls isotonische Zusätze enthalten, um deren osmotischen Druck zu erhöhen. Geeignete Zusätze sind beispielsweise anorganische oder organische Salze oder Puffersubstanzen, wie Natriumchlorid, Phosphat-Puffer. Citrat-Puffer, Glycin-Puffer, Citrat-Phosphat-Puffer, Maleat-Puffer, etc. Mono- oder Disaccharide. wie Glucose, Lactose, Saccharose. Zuckeralkohole, wie Mannit, Sorbit, Xylit oder Glycerin oder wasserlösliche Polymere, wie Dextran oder Polyethylenglykol.

Diese isotonisierenden Substanzen werden üblicherweise in solchen Konzentrationen zugesetzt, daß die entstehende wässrige Mischmicellösung einen osmotischen Druck von 5 - 1000 mosm - bei Injektionslösungen optimalerweise 300 mosm - aufweist.

Die Herstellung der wasserlöslichen Mischmicellösungen erfolgt - abgesehen von der Membrandestillation mittels konventioneller Methoden.

Da die Lipide und auch einige Wirkstoffe oxidationsempfindlich sind, wird das Verfahren zweckmäßigerweise unter einer Inertgasatmosphäre, wie Stickstoff oder Argon durchgeführt und die erhaltenen wässrigen Mischmicellösungen durch Zugabe von Antioxidantien, wie Natriumascorbat, Tocopherol oder Natriumhydrogensulfit stabilisiert.

Diese Mischmicellösungen können beispielsweise zur Solubilisierung der bereits erwähnten schwer löslichen Wirkstoffe verwendet werden.

Ferner können die wässrigen Mischmicellösungen noch zusätzliche wasserlösliche Wirkstoffe enthalten, um Kombinationspräparate herzustellen. Beispiele solcher Kombinationspräparate sind Mischungen aus wasserlöslichen und fettlöslichen Vitaminen oder Präparate die neben Kortikoiden noch wasserlösliche Antibiotika enthalten.

Zur Herstellung liposomenhaltiger, wässriger Phasengemische verwendet man vorzugsweise die bereits erwähnten Phospholipide und Gemische dieser Phospholipide mit Cholesterin und/oder Ladungsträgern wie zum Beispiel Stearylamin, Stearinsäure oder Dicetylphosphat. Hierbei werden vorzugsweise 0,1 bis 40 Gewichtsprozent und insbesondere 1 bis 20 Gewichtsprozent Phospholipid oder Gemisch bezogen auf die die wässrige Phase verwendet. Geeignete Gemische enthalten etwa bis zu 60 Gewichtsprozent Cholesterin und bis zu 15 Gewichtsprozent Ladungsträger. Als Lösungsmittel für die Phospholipide oder Gemische verwendet man vorzugsweise Methanol, Ethanol, Isopropanol, Diethylether, Dioxan, Aceton, Chloroform, Acetonitril, Dimethylsulfoxid und Gemische dieser Lösungsmittel.

Das erfindungsgemäße Verfahren wird abgesehen von der Membrandestillation unter den gleichen Bedingungen durchgeführt, wie die vorbekannten Verfahren (Pharmazie in unserer Zeit 11, 1982, 97-108, Pure Appl. Chem., 53, 1981, 2241-2254). Es eignet sich sowohl zur Herstellung multilamellarer Liposomen als auch zur Herstellung unilamellarer Liposomen und ist besonders zur Herstellung großer unilamellarer Liposomen mittels der reverse-phase-evaporation geeignet.

Die liposomenhaltigen, wässrigen Phasengemische können die gleichen Zusätze enthalten, wie die Mischmicellösungen und können beispielsweise zur Verkapselung wasserlöslicher Wirkstoffe dienen.

Solche wasserlöslichen Wirkstoffe sind beispielsweise Diagnostika, wie die Röntgenkontrastmittel Iotrolan, Iohexpl, Iosimid, Metrizamid, Salze von Amidoessigsäure und insbesondere Iopromid oder NMR-Kontrastmittel, wie das Gadolinium-DTPA.

Geeignete Arzneimittelwirkstoffe sind unter anderen Antibiotika, wie Gentamycin oder Kanamycin, Cytostatica, wie Doxorubicin-Hydrochlorid oder Cyclophosphamid und Virustatica, wie Vidarabin.

Darüberhinaus können die liposomenhaltigen, wässrigen Phasen auch zur Verkapselung der bereits erwähnten in Wasser schwer löslichen Wirkstoffe verwendet werden.

Die nachfolgenden Ausführungsbeispiele dienen zur näheren Erläuterung des erfindungsgemäßen Verfahrens.

### Beispiel 1

12 g Phosphatidylcholin werden in 500 ml Diethylether gelöst, und diese Lösung in einen Kolben überführt, der mit 500 ml einer Wirkstofflösung beschickt ist, die 400 mg Iotrolan pro ml enthält. Dieses Gemisch wird mit einem Hochdruckhomogenisator homogenisiert, wobei eine Art Wasser in Ol Emulsion entsteht, in der die Wasserphase in Form von "inversen Micellen" dispergiert vorliegt. Diese Emulsion wird anschließend mittels einer Zahnradpumpe im Kreislauf durch zwei in Serie geschaltete Filtrationsmodule gefördert, welche aus jeweils drei Polypropylen-Rohrmembranen aufgebaut sind ("Labormodul" der Firma Enka AG, DE-5600 Wuppertal). An die Außenseite der Membran wird hierbei ein Druck von 8000 Pa mittels einer Evakuierungseinrichtung angelegt. Während der Entfernung des Lösungsmittels wird das Vorratsgefäß, welches einen temperierbaren Mantel aufweist, bei Temperaturen zwischen 30 und 35° C gehalten.

Die Anfangsdurchflußgeschwindigkeit liegt bei 6 l/min und der daraus resultierende Moduleingangsdruck bei 70 000 Pa. Mittels eines Manometers und eines Durchflußmessers werden diese beiden Parameter während der Lösungsmittelentfernung beobachtet. Nach bereits 15 Minuten bilden sich erste Gel-Strukturen, die während der Abtrennung mehrmals von den Wänden des Vorratsgefäßes abgestreift werden. Die Durchflußgeschwindigkeit nimmt im Laufe der weiteren Abtrennung ab, wobei ein Anstieg des Moduleingangsdruckes zu verzeichnen ist. Nach 50 Minuten bildet sich ein viskoses Gel, wodurch der laut Herstellerangaben maximal zulässige Moduleingangsdruck von 150 000 Pa überschritten wird. Der Ethergehalt liegt an diesem Punkt bei 5-10%. Portionsweise Zugabe von 150 ml Pufferlösung (0.015 M Tris-HCl Puffer pH-Wert 7,4) ) führt dazu, daß das Gel bricht und eine wässrige Liposomensuspension entsteht. Durch weitere Membrandestillation im Kreislauf läßt sich die Etherkonzentration auf unter 2 % absenken.

### Beispiel 2

12 g Phosphatidylcholin werden in 125 ml Ethanol gelöst und diese Lösung in einen Kolben überführt der mit 500 ml einer wässrigen Wirkstofflösung beschickt ist. die 400 mg Iotrolan pro ml enthält. Anschließend wird die Mischung durch Schütteln vermischt.

Dieses Gemisch wird danach mittels einer Zahnradpumpe im Kreislauf durch zwei in Serie geschaltete Filtrationsmodule gefördert, welche aus jeweils drei Polypropylen-Rohrmembranen aufgebaut sind ("Labormodul" der Firma Enka AG, DE-5600 Wuppertal).

An der Außenseite der Membran wird hier bei im Gegenstrom 1000 ml destilliertes Wasser, welches mittels einer Methanol-Trockeneismischung gekühlt wird (5-10° C) entlanggeführt. Während der Entfernung des Lösungsmittels wird das Vorratsgefäß, welches einen temperierbaren Mantel aufweist, bei Temperaturen um 35° C gehalten.

Die Anfangsdurchflußgeschwindigkeit liegt bei 5 l/min und der daraus resultierende Moduleingangsdruck bei 50 000 Pa. Mittels eines Manometers und eines Durchflußmengenmessers werden diese beiden Parameter während der Lösungsmittelentfernung beobachtet. Nach ca. 2 Stunden erhält man eine weißliche Liposomensuspension. Der mittlere Durchmesser der hierin enthaltenen Liposomen liegt bei 261,7 ± 5 nm. Der Iotrolaneinschluß beträgt 25 mg Iotrolan/ml und der Restethanolgehalt liegt bei unter 1 %.

### Beispiel 3

32,4 g eines Gemisches aus Phosphatidylcholin, Cholesterin und Stearinsäure (4:5:1) werden bei erhöhter Temperatur in 350 ml Ethanol gelöst. Diese Lösung wird unter Rühren in einen Kolben überführt der 700 ml einer wässrigen Lösung von 32,4 g Iopromid in 0,020 Tris HCl Puffer (pH Wert 7,5) enthält. Die Abtrennung des Ethanols erfolgt anschließend wie in Beispiel 2 beschrieben. Abweichend von dem vorgenannten Beispiel wird das Vorratsgefäß jedoch auf 55 Grad Celsius termperiert.

Nach ca. 5 Stunden erhält man auch hier weißliche Liposomensuspensionen. Der mittlere Durchmesser der darin enthaltenen Liposomen liegt bei 370 nm und der Iopromideinschluß bei 36 % bezogen auf die Gesamte Kontrastmittelkonzentration. Der Restbethanolgehalt beträgt weniger als 0,1 %.

## Patentansprüche

1. Verfahren zur Herstellung einer Liposomen oder Micellen enthaltenden wässrigen Dispersion, indem man die Liposomen und/oder Micellen bildenden Substanzen und gegebenenfalls in Wasser schwer lösliche oder unlösliche Wirkstoffe in einem organischen Lösungsmittel löst, die Lösung in eine wässrige Phase einträgt und anschließend das organische Lösungsmittel destillativ entfernt, welches dadurch gekennzeichnet ist, daß die destillative Entfernung des organischen Lösungsmittels mittels Membrandestillation erfolgt.

2. Verfahren zur Herstellung einer Liposomen oder Micellen enthaltenden wässrigen Dispersion gemäß Patentanspruch 1, dadurch gekennzeichnet, daß das zu entfernende Lösungsmittel ein solches mit einem Siedepunkt von maximal 300°C ist.

3. Verfahren zur Herstellung von Liposomen oder Micellen enthaltenden wässrigen Dispersionen gemäß Patentanspruch 1 und 2, dadurch gekennzeichnet, daß man das Lösungsmittel mit transmembraner Destillation oder Pervaporation entfernt.

## Claims

1. Process for the preparation of an aqueous liposome- or micelle-containing dispersion, wherein the liposome- and/or micelle-forming substances and, optionally, active substances that are sparingly soluble or insoluble in water are dissolved in an organic solvent, the solution is introduced into an aqueous phase and the organic solvent is then removed by distillation, characterised in that the removal of the organic solvent by distillation is carried out by means of membrane distillation.

2. Process for the preparation of an aqueous liposome- or micelle-containing dispersion according to patent claim 1, characterised in that the solvent to be removed is one having a boiling point of a maximum of 300°C.

3. Process for the preparation of aqueous liposome- or micelle-containing dispersions according to patent claims 1 and 2, characterised in that the solvent is removed by transmembrane distillation or pervaporation.

## Revendications

1. Procédé de préparation d'une dispersion aqueuse contenant des liposomes ou des micelles, selon lequel on dissout dans un solvant organique les substances formatrices de liposomes et/ou de micelles et éventuellement des substances actives peu solubles ou insolubles dans l'eau, on introduit la solution dans une phase aqueuse et l'on en élimine par la suite le solvant organique par distillation, procédé caractérisé en ce que l'élimination par distillation du solvant organique a lieu à l'aide d'une distillation sur membrane.

2. Procédé de préparation d'une dispersion aqueuse contenant des liposomes ou des micelles selon la revendication 1, procédé caractérisé en ce que le solvant à éliminer est un solvant présentant un point d'ébullition d'au maximum 300°C.

3. Procédé de préparation de dispersions aqueuses contenant des liposomes ou des micelles selon la revendication 1 et la revendication 2, procédé caractérisé en ce qu'on élimine le solvant par distillation transmembranaire ou par pervaporation.
